# DEMANDE DE BREVET EUROPEEN

(11) **EP 2 695 584 A1**
(43) Date de publication de la demande: **12.02.2014**
(21) Numéro de dépôt: 13384201.3
(22) Date de dépôt: 19.06.2013
(51) Int. Cl.: A61F 2/00

(54) **Dispositif fixateur et ocluseur du pénis pour l'incontinence urinaire.**

(30) Priorité: 10.08.2012 ES 201200849
(71) Demandeur: Garcia Berruezo, Pedro, 29740 Torre del Mar (Málaga) (ES); Garcia Almanzor, Estefania, 29740 Torre del Mar (Málaga) (ES)
(72) Inventeur: Garcia Berruezo, Pedro, 29740 Torre del Mar (Málaga) (ES); Garcia Almanzor, Estefania, 29740 Torre del Mar (Málaga) (ES)

(57) **Abrégé**

Le dipositif fixateur et ocluseur du pénis pour l'incontinence urinaire est un anneau aplati d'un matériau souple qui recouvre une structure métatique intérieure. Il est déformable, réglable et dispose de deux grossissements (5) semi-circulaires et un grossissementt allongé au bas inférieure(6).

## Description

### SECTEUR DE LA TECHNIQUE

Il s'agit d'un dispositif médical qui s'inscrit dans l'industrie médicale orthopédique, appartient précisément à la branche de l'urologie.

### ANTÉCÉDENTS DE L'INVENTION

Le dispositif fixateur et ocluseur du pénis pour l'incontinence urinaire, pour lequel ce brevet est demandé, apparaît comme une solution au problème des dispositifs existants:
1) Ceux qui sont fermés avec du ruban Velcro, il faut les démonter à chaque fois que l'on urine, ils peuvent se tourner autour du pénis et en conséquence ils perdent la pression sur l'urètre.
2) Ceux qui ont la forme de pince, dans lesquels le pénis est déplacé latéralement et également il se produit des sorties involontaires d'urine.

Ce sont les brevets antérieurs qui sont liés à ce dispositif:
ES P200802479 (PEDRO GARCÍA BERRUEZO) 28-02-2012.
WO 2007130977 A1 (GT UROLOGICAL LLC et al.) De 15-11-2007, page 4, lignes 14-18, page 8, lignes 15-22, à la page 22, ligne 26, à la page 23, ligne 3, page 25, ligne 1-13; 9A-11B.
WO 2005110279 A2 (Novatek MEDICAL LLC; TIMM et al.) De 24-11-2005, page 3, lignes 14-30, page 4, lignes 6-14, page 5, lignes 15-17, page 9, lignes 12 - 21, page 14, ligne 7, page 16, ligne 16, les figures 8a-9.
DE 10213452 C1 (YONYE ADAM) 17-07-2003, résumé; chiffres. US 6349727 B1 (POS-T-VAC INC). 26-02-2002, colonne 1, ligne 43, colonne 2, ligne 16, colonne 4, lignes 34-59, fig.
DE 20112864 U1 (Urovision GMBH) 22-11-2001, résumé; chiffres. US 3147754 A (KOESSLER) 08/09/1964, colonne 3, lignes 30-50, fig. US 714 850 A (ZIMMERMANN) 12/02/1902, lignes 10-48, fig.

### DESCRIPTION DE L'INVENTION

### A) DESCRIPTION BRÈVE

Il consiste d'une structure métallique flexible recouverte d'un matériau souple sur-injecté (thermoplastique ou de caoutchouc de silicone) avec deux grossissements allongés semi-circulaires sur la position supérieure qui centre et immobilise le pénis. Ces grossissements supérieurs provoquent que la pression exercée par le grossissement allongé inférieur sur l'urètre soit beaucoup plus efficace dans le contrôle de l'incontinence urinaire.

### B) DESCRIPTION DÉTAILLÉE DE L'INVENTION

Le dispositif fixateur et ocluseur du pénis pour l'incontinence urinaire est un anneau aplati d'un matériau souple qui lorsqu'il est pressé latéralement devient ovale.

Sa structure interne est formée par deux bandes incurvées d'acier et articulée au niveau des extrémités par deux charnières avec les repères qui permettent le changement d'une forme aplatie à une ovale par une simple pression latérale avec deux doigts.

La structure globale de métal a un bain électrolytique d'inoxydable.

Il dispose aussi deux chambres latéraux libres de pression qui permettent le passage du flux sanguin.

Pour la régulation de pression requisé par chaque patient il présente deux trous dans lesquels sont insértées les petits cylindres de diférents diamètres.

### EXPLICATION DÉTAILLÉE DES DESSINS.

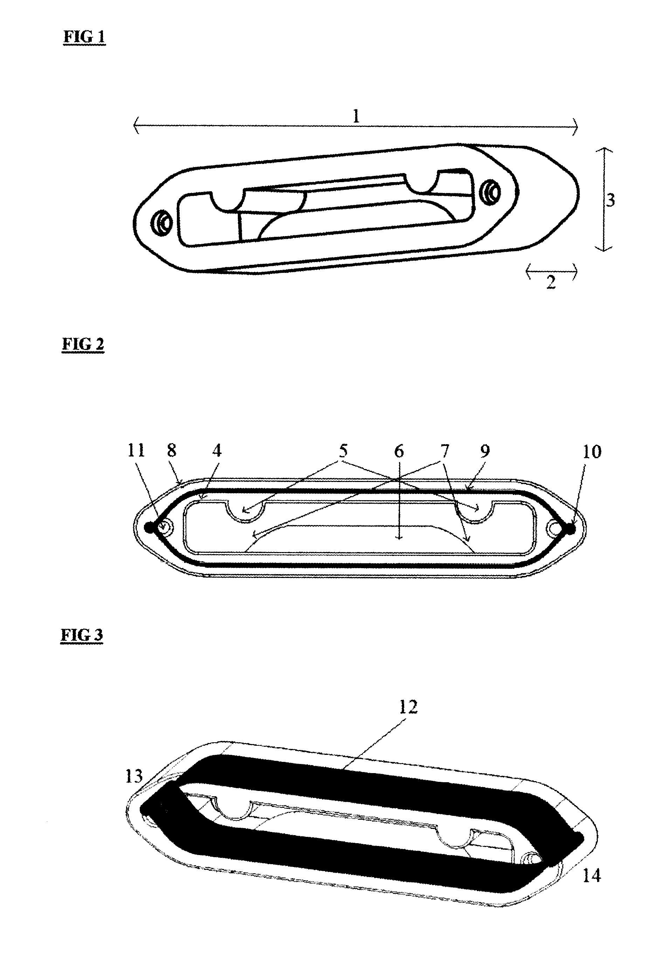

La FIG 1 c'est le dessin par rapport au plan inférieur dans lequel le n°1 c'est la longueur totale de 91 mm, le n°2 c'est la largeur de 20 mm et le n°3 c'est la hauteur de 20 mm.

La FIG 2 c'est la vue de face du dispositif dans lequel la structure métallique est représentée internement. Le n° 4 c'est l'épaisseur de la matière souple sur-injectée, qui est de 5 mm, avec tous les bords arrondis, comme le n° 8 indique. Le n°.5 ceux sont les deux demi-cercles supérieurs qui vont centrer et immoviliser le pénis, ils ont 4 mm de diamètre, 20 mm de long et la distance entre leurs centres c'est 46 mm. Le n ° 6 c'est le grossissement inférieur semi-circulaire d'une longueur égale à l'espace supérieur pour faire pression optimale sur l'urètre, sa longueur est de 46 mm et une hauteur de 5,5 mm, laissant un espace de 4,5 mm vers le haut. Le n° 7 correspond à deux espaces latéraux qui facilitent la circulation constante en glissant la peau du pénis. Le n° 9 c'est la forme de la structure métallique interne . Le n°10 ceux sont les articulations de la charnière de la structure métallique pour faciliter que le dispositif peut être transformé en ovale. Le n° 11 ceux sont des trous de 2,5 mm de diamètre, pour que chaque patient place les paires de cilindres de 4, 5 ou 6 mm de diamètre et 15 mm de long pour donner plus ou moins pression sur urètre.

La FIG 3 c'est une autre vue en perspective de la partie supérieure dans laquelle on observe comment la structure métallique est disposée à l'intérieur et le revêtement souple avec des bords arrondis. Le n ° 12 c'est la structure métallique interne constituée par deux bandes incurvées en acier de 15 mm de largeur et 0,25 mm d'épaisseur, qui est articulée au niveau des extrémités par deux articulations de charnière. A l'intérieur, la distance longitudinale entre les deux articulations est de 80 mm, la hauteur centrale 15, 5 mm. La distance entre les deux trous pour les régulateurs est de 77,5 mm. Les numéros 13 et 14 indiquent l'endroit où l'on place les deux doigts pour appuyer vers l'intérieur et qu'il se transforme en une forme ovale.

### EXEMPLE DE REALISATION DE L'INVENTION

Le dispositif fixateur et ocluseur du pénis pour l'incontinence urinaire comprend une structure métallique interne constituée par deux bandes en acier qui sont articulées au niveau des extrémitées par deux charnières . Ces bandes, comme il a été mentionné dans la description des dessins, ont 15 mm de large par 0,25 d'épaisseur et elles sont articulées à une distance de 80 mm. Cette structure métallique est recouverte du nickel électrolytique d'épaisseur, 10 microns, pour la convertir en inoxydable.

Cette structure porte un revêtement de matière thermoplastique sur-injecté, ou de caoutchouc de silicone médical certification USP Classe VI (pour être en contact avec la peau) ayant une épaisseur de 5 mm pour les parties les plus fines, ce qui donne au dispositif une sécurité absolue qui évite, dans une hypothétique rupture de la structure métalique, la sortie de celle-ci.

Dans l'explication des dessins il y a absolument toutes les mesures du dispositif.

Notez que tous les bords sont arrondis pour éviter l'inconfort, ce qui n'arrive pas avec celles qui existent déjà.

Pour un procédé de fabrication devrait garder à l'esprit les éléments suivants:
1° technologie 3D par Cad
2° Le développement d'un moule, en deux moitiés, les mâles faisant des trous et, à son tour tenir la structure métallique dans la sur-injection.
3° Placer les structures à l'intérieur du moule.
4° Sur-injection d'un matériau souple.
5° Démoulage des pièces.

## Revendications

1. Le dispositif fixateur et ocluseur du pénis pour l'incontinence urinaire est formé de deux bandes en acier inoxydable,elles sont déformables, articulées et revêtues d'un matériau souple avec deux trous pour réguler la pression et **il est caractérisé par** deux grossissements semi-circulaires allongés dans la partie supérieure intérieure.

## Revendications modifiées

### Revendications modifiées conformément à la règle 137(2) CBE.

**1.** Le dispositif fixateur et occluseur du pénis pour l'incontinence urinaire se caractérise parce-que, pour uriner, il ne faut pas le dévisser, retirer ou démonter.

**2.** Le dispositif fixateur et occluseur du pénis pour l'incontinence urinaire se caractérise parce-que, après avoir uriné, et quand la pression latérale des doigts sur le dispositif cesse, ce dispositif lui-même tourne à sa position initiale, à la pression prédéfinie et à centrer le pénis grâce aux deux régulateurs et aux deux grossissements semi-circulaires supérieurs.
